# EUROPÄISCHE PATENTSCHRIFT

(11) **EP 1 881 973 B1**
(45) Veröffentlichungstag und Bekanntmachung des Hinweises auf die Patenterteilung: **11.07.2012**
(21) Anmeldenummer: 06753482.6
(22) Anmeldetag: 04.05.2006
(51) Int. Cl.: C07D 317/40

(54) **VERFAHREN ZUR HERSTELLUNG VON VINYLENCARBONAT**
PROCESS FOR PRODUCING VINYLENE CARBONATE
PROCEDE DE PRODUCTION DE CARBONATE DE VINYLENE

(30) Priorität: 12.05.2005 DE 102005021964
(43) Veröffentlichungstag der Anmeldung: 30.01.2008
(73) Patentinhaber: Saltigo GmbH, 40764 Langenfeld (DE)
(72) Erfinder: LANGER, Reinhard, 47918 Tönisvorst (DE); BECKMANN, Anke, 51067 Köln (DE); SCHULZE TILLING, Andreas, 42799 Leichlingen (DE); WAGNER, Paul, 40597 Düsseldorf (DE); MLECZKO, Leslaw, 41542 Dormagen (DE); BUCHHOLZ, Sigurd, 50825 Köln (DE)
(74) Vertreter: Pettrich, Klaus-Günter
(86) Internationale Anmeldenummer: PCT/EP2006/004155
(87) Internationale Veröffentlichungsnummer: WO 2006/119909

(56) Entgegenhaltungen:
- US-A- 2 918 478
- US-A- 3 030 382
- US-A- 3 156 702
- PATENT ABSTRACTS OF JAPAN Bd. 2002, Nr. 11, 6. November 2002 (2002-11-06) & JP 2002 193892 A (UBE IND LTD), 10. Juli 2002 (2002-07-10)

## Beschreibung

Die vorliegende Erfindung betrifft die technische Herstellung von Vinylencarbonat (VC) durch Abspaltung von Chlorwasserstoff aus Chlorethylenglykolcarbonat (CGC) an festen Kontakten in der Gasphase.

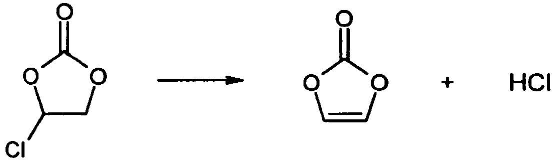

Vinylencarbonat ist ein wichtiges Zwischenprodukt für die Herstellung von Chemikalien, Pharmaprodukten, Pflanzenschutzmitteln und im Besonderen für Polymere, Lacke und Batterieelektrolyte.

Vinylencarbonat wird nach bekannter Methodik durch Abspalten von Chlorwasserstoff aus Chlorethylenglykolcarbonat mittels tertiärer Amine, insbesondere Triethylamin hergestellt.

Das Chlorethylenglykolcarbonat gewinnt man durch radikalische Chlorierung von Ethylenglykolcarbonat mittels Chlor oder Sulfurylchlorid.

Erstmals veröffentlicht wurde diese Synthese 1953 von Newman und Addor (JACS, 1953, S. 1263; JACS 1955, S. 3789).

Ethylenglykolcarbonat (GC) wurde mittels ultraviolettem Licht bei 60-70°C in Substanz photochloriert und das entstandene CGC durch Vakuumdestillation gereinigt.

VC erhielten Newman und Addor durch Eliminierung mittel Triethylamin in siedendem Ether, wobei das Gemisch über Nacht erhitzt wurde.

Die Isolierung erfolgte durch Abfiltrieren des Triethylammoniumchlorides und anschließende Destillation, die mit 59%iger Ausbeute ein rohes VC lieferte, welches durch weitere Destillation gereinigt werden musste.

US 3 156 702 A offenbart einen Prozess zur Herstellung von Vinylencarbonat aus Chlorethylenglykolcarbonat and festen Kontakten in der Gasphase bei einer Temperatur von 300-400°C, wobei die festen Kontakte Salze und Oxide der I., II. und VIII. Nebengruppe des PSE sein können d.h. z.B. Kupfer, Zink, Nickel, Kobalt, Cadmium und Eisen bzw. deren Halogenide.

JP 2000/026449 beschreibt die Eliminierung in hochsiedenden Lösungsmitteln (Sdp. 170-300°C), explizit wird mit Triethylamin in Dibutylcarbonat 20 Stunden bei 50°C umgesetzt.

Nach dem Abfiltrieren des Ammoniumchlorides und dem Abdestillieren von überschüssigem Triethylamin wird rohes VC durch einfache Destillation isoliert. Um Spuren von Aminen zu entfernen wird das VC über eine Silicagelsäule gegeben. Abschließend wird eine Feindestillation durchgeführt. Der Chlorgehalt des so gewonnenen VC wird mit 29 ppm angegeben, während Vergleichsproben >3000 ppm enthalten. Die Ausbeute beträgt 56%.

DE-A 19 955 944 claimed die Eliminierung in GC als Lösungsmittel (Sdp. 243-244°C). CGC wird in GC vorgelegt und in 1,5 Stunden durch Zugabe von Triethylamin bei 60°C umgesetzt. Nach Abdestillieren von überschüssigem Triethylamin bei 40°C und Verdampfen über einen Dünnschichter bei 100°C wird mit 73%iger Ausbeute ein farbloses Gemisch aus VC und GC gewonnen. Über die Reinheit werden keine Angaben gemacht.

Die Umsetzungen von CGC in flüssiger Phase leiden alle unter der Zersetzlichkeit des VC, welche in DE 199 55 944 A1 explizit angesprochen wird. Oberhalb von 60°C zersetzt es sich demnach in Stunden und oberhalb von 80°C in Minuten. Die dabei entstehenden Polymere erschweren das Absaugen der Salze und die exotherme Zersetzung macht das scale up solcher Prozesse problematisch.

Johnson und Patton beschrieben im JOC, 1960, S. 1042 die Umsetzung von CGC an festen Schüttungen von CaSO₄-Katalysatoren in der Gasphase bei 250°C und 6.67-8 kPa (50-60 Torr).

Die Katalysatoren deaktivieren sehr rasch und erreichen bestenfalls einem Umsatz von 35-40%, bei einer Selektivität von 40-45%. Höhere oder niedrigere Temperaturen führen zu weniger Umsatz. Die Katalysatoren können durch Abbrennen regeneriert werden.

Granulierte Aktivkohle und granuliertes aktiviertes Aluminiumoxid liefern nur gasförmige Produkte.

DE-A 1 135 452 beschreibt die HCl-Abspaltung von CGC bei 300-400°C. Das CGC wird gasförmig über ein inertes Trägermaterials geleitet, welches mit Elementen der I., II. oder VIII. Nebengruppe des Periodensystem bzw. deren Salzen oder Oxyden belegt ist. Vorzugsweise werden die Chloride des Eisens, Kobalts, Kupfers, besonders bevorzugt Cadmiumchlorid eingesetzt. Geeignete Trägermaterialien sind Bimse und Silicate mit Körnungen von 4 bis 8 mm.

Betrieben werden die Katalysatoren als stationäre Schüttung bei Normaldruck oder Unterdruck und einer Temperatur von 270 bis 450, bevorzugt von 300-400°C.

Das Verhalten von CdCl₂ auf Bimsstein wird explizit beschrieben. Der Katalysator ist mit ca. 270 Stunden deutlich standfester und mit 74% selektiver als die CaSO₄-Kontakte.

Die Belastung betrug 0,15 kg CGC pro L Katalysator und Stunde und der Inertgasstrom lag zwischen 27 bis 67 L pro kg CGC. Der Durchschnittliche Umsatz betrug 87%.

Der Katalysator kann bei 500 bis 700°C mit Luft abgebrannt werden.

Unbefriedigend bei den Gasphasenverfahren ist der trotz niedriger Belastung geringe Umsatz, ferner der Einsatz von giftigem CdCl₂. Das Trägermaterial Bimsstein ist sehr weich und mechanisch empfindlich. Besonders problematisch an einem solchen Verfahren ist jedoch, dass starke Ablagerungen von kohlenstoffreichen Verbindungen den regelmäßig durchzuführenden Abbrennvorgang schwer beherrschbar machen. Ein Scale Up in den technischen Maßstab ist daher schwierig und sehr riskant. Die Standfestigkeit solcher Katalysatoren über viele Regenerationszyklen ist ebenfalls völlig offen.

Aufgabe der Erfindung ist daher die Bereitstellung eines Gasphasenverfahrens, das höhere Umsätze und Selektivitäten liefert und bei höheren Belastungen und ggf. geringerem Inertgasstrom eine sichere und einfache Handhabung aller Betriebszustände im technischen Maßstab ermöglicht, wobei giftige Schwermetalle wie Cd nach Möglichkeit zu vermeiden sind.

Die Tatsache, dass seit 1953 etliche Veröffentlichungen zur nasschemischen Eliminierung mit Triethylamin erschienen sind, zur Gasphaseneliminierung aber nur der Artikel von Johnson und Patton von 1960 und die DE-A-1 135 452 von 1961 existieren, weist darauf hin, dass die Schwierigkeiten der Eliminierung von HCl aus CGC in der Gasphasen an heterogenen Kontakten in der Fachwelt als unüberwindbar eingestuft waren.

Überraschenderweise wurde gefunden, dass die gesuchten Verfahrenseigenschaften erzielt werden, wenn man mit durch Durchmischung bewegten Katalysatorschüttungen bzw. mit Wirbelschichten arbeitet. Als Aktivkomponente auf inertem Trägermaterial ist Zinkchlorid besonders geeignet, was überrascht, da Zinkchlorid bereits bei 290°C schmilzt.

Ein Verfahren zur Herstellung von Vinylencarbonat durch Abspaltung von Chlorwasserstoff aus Chlorethylenglykolcarbonat (CGC) an festen Kontakten in der Gasphase, dadurch gekennzeichnet, dass die Umsetzung zwischen 300 und 600°C an durch Durchmischung bewegten Katalysatorschüttungen gegebenenfalls unter Inertgas, durchgeführt wird..

Das erfindungsgemäße Verfahren kann in verschiedenen Reaktortypen durchgeführt werden, z.B. Reaktoren mit durchmischten Katalysatorschüttungen, wie Schaufeltrocknern, Drehrohröfen, bevorzugt mit einem blasenbildenden, turbulenten oder durchstrahlten Wirbelbett, intern oder extern zirkulierende Wirbelbetten, Wirbelbetten mit bewegten Einbauten oder zusätzlichen Blasenteilern, Reaktoren mit einer mechanischen oder zusätzlichen Agitation des Bettes zum Beispiel durch Schall oder Klopfer/Schwingungen wie z.B. Schall oder Ultraschall. Bevorzugt wird es in einer gerührten blasenbildenden Wirbelschicht durchgeführt.

Als gegebenenfalls zugesetzte Inertgase kommen alle Gase in Frage, die unter den gewählten Reaktionsbedingungen nicht mit dem Edukt oder Produkt reagieren, besonders geeignete Inertgase sind Edelgase wie Argon, Helium und Neon, Stickstoff, Kohlenmonoxid oder Kohlendioxid oder Halogenwasserstoffverbindungen wie HCl. Bevorzugt werden als Inertgase Gase aus der Gruppe Stickstoff, Argon, Kohlenmonoxid oder Kohlendioxid eingesetzt. Es ist möglich, das erfindungsgemäße Verfahren unter Zugabe eines inerten Gases oder einer Mischung mehrerer inerter Gase in beliebiger Kombination durchzuführen. Es kann auch vollständig auf ein Trägergas verzichtet werden.

Die Temperatur kann im Temperaturbereich von 300°C bis 600°C variiert werden. Der vorteilhafte Temperaturbereich liegt zwischen 350°C und 500°C. Bevorzugt ist eine Reaktionstemperatur von 380°C bis 430°C.

Für das erfindungsgemäße Verfahren werden im Reaktionsraum Feststoffpartikel des Kontaktes eingebracht. Die Partikel bilden ein Festbett, durch das das zugeführte Gas eingeströmt wird. Die Einströmgeschwindigkeit des zugeführten Gases kann so eingestellt werden, dass das Festbett fluidisiert wird und sich eine Wirbelschicht ausbildet. Die entsprechende Vorgehensweise ist dem Fachmann an und für sich bekannt. Die Einströmgeschwindigkeit des zugeführten Gases muss dazu mindestens der Lockerungsgeschwindigkeit (Minimumfluidisierungsgeschwindigkeit) entsprechen. Unter Lockerungsgeschwindigkeit wird dabei die Geschwindigkeit verstanden, mit der ein Gas durch ein Bett aus Partikeln strömt und unterhalb derer das Festbett erhalten bleibt, d.h. unterhalb derer die Bettpartikel weitgehend unbewegt bleiben. Oberhalb dieser Geschwindigkeit beginnt die Fluidisierung des Betts, d.h. die Bettpartikel bewegen sich und es bilden sich erste Blasen. Beim Betrieb einer blasenbildenden Wirbelschicht wird die Gasgeschwindigkeit so gewählt, dass die dem ein bis fünfzigfachen der Lockerungsgeschwindigkeit entspricht, bevorzugt dem ein bis vierzigfachen, besonders bevorzugt dem ein- bis dreißigfachen.

Der feste Kontakt besteht aus einem inerten Trägermaterial mit aufgebrachten Metallen der I., II. oder VIII. Nebengruppe des Periodensystems der Elemente, deren Salze oder Oxyde, bevorzugt werden Chloride eingesetzt, besonders bevorzugt Zinkchlorid.

Als Trägermaterial sind Silicate mit BET-Oberfläche kleiner 10, bevorzugt kleiner 1, besonders bevorzugt kleiner 0,1 m²/g geeignet, bevorzugt poröse oder raue Gläser.

Ferner kann der feste Kontakt ganz aus Salzen oder Oxyden der Metalle der I., II. oder VIII. Nebengruppe des Periodensystems der Elemente ohne Trägermaterial bestehen, d.h. es kann auf einen Träger verzichtet werden, bevorzugt werden Oxyde und/oder Chloride eingesetzt.

Desaktivierter Kontakt kann durch Abbrennen mit Luft bzw. Gemischen aus Sauerstoff und inertem Gas bei Temperaturen zwischen 300 und 700°C, bevorzugt zwischen 400 und 600°C regeneriert werden.

Verlorene Aktivkomponenten können durch Tränken oder Aufsprühen von z.B. wässrigen Lösungen der Salze ersetzt werden.

Die Abtrennung von feinteiligem festem Austrag aus dem den Reaktor verlassenden Gasstrom kann beispielsweise über einen Zyklon, einen Filter oder über einen Gaswäscher erfolgen. Bevorzugt ist eine Abtrennung über einen Zyklon und/oder einen Filter. Das abgetrennte Material kann durch eine geeignete Rückführung direkt in das Bett zurückgeführt werden oder getrennt vom Produkt gesammelt werden.

Im Folgenden wird das erfindungsgemäße Verfahren anhand einiger Beispiele illustriert, wobei die Beispiele jedoch nicht als Einschränkung des Erfindungsgedankens zu verstehen sind.

### Beispiele:

### Beispiel 1 (Katalysatorherstellung für Rohrreaktor):

2L = 1052g eines porösen Siliziumdioxidträgers mit einer Kugelgröße zwischen 1 und 2mm wurden mit 400 ml Tränkflüssigkeit so lange bei Raumtemperatur bewegt, bis alle Tränkflüssigkeit aufgenommen worden ist.

Die Tränkflüssigkeit bestand aus 80g Zinkchlorid und Wasser.

Anschließend wurde der Katalysator bei 110°C im Trockenschrank getrocknet und abschließend bei 400°C 3 h calziniert.

Das Trägermaterial besaß eine scheinbare Dichte von 1,06 g/ml. Die Hg-Porosimetrie ergab ein penetriertes Volumen von 524 mm³/g. Damit betrug die Porosität ca. 56%. Die BET-Oberfläche, gemessen durch Stickstoffadsorbtion bei -196°C war < 0,05 m²/g. Die spezifische Oberfläche berechnet aus der Hg-Verteilung war ca. 0,4 m²/g.

Die Oberflächenzusammensetzung, bestimmt durch XPS, angegeben in Atom%, 5,2% Mg, 11 % Na, 1,9% F, 54,6% O, 1,3% Cl und 26% Si. Das Volumen enthielt 3,1% Mg, 11,5% Na, 1,1% F, 53,4% O, 2,9% Ca, 1% Cl und 27% Si.

### Beispiel 2 (Katalysatorherstellung für Wirbelbettreaktor):

Der in Beispiel 1 beschriebene Träger wurde in einem Frewittsieb zerkleinert und mit einer Analysensiebmaschine die Fraktion 0,315-0,16mm abgetrennt.

1250 ml = 1029g wurden mit 597 ml Tränkflüssigkeit bestehend aus 50g Zinkchlorid und Wasser bei Raumtemperatur bewegt, bis alle Tränkflüssigkeit aufgesaugt worden war.

Anschließend wurde 24 h bei 110°C und 200 mbar getrocknet.

### Beispiel 3 (Wirbelbettversuch):

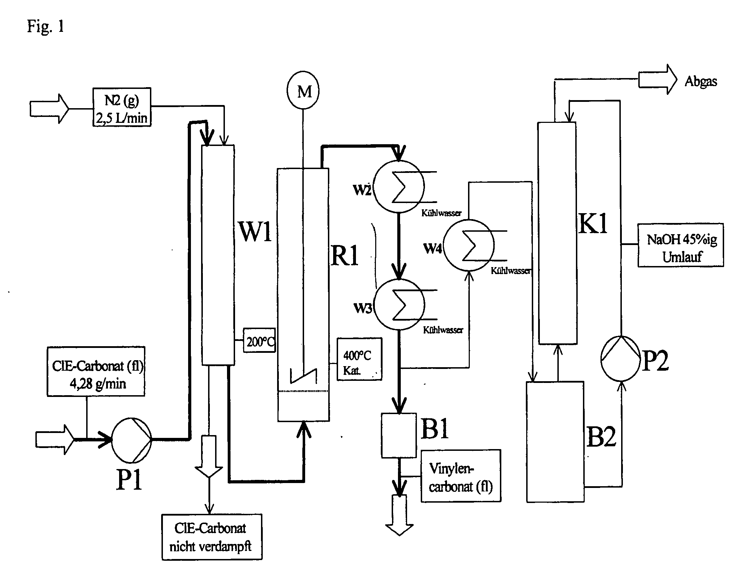

Die Apparatur ist in der Fig. 1 dargestellt.

Für die Versuchsdurchführung wurde ein Quarzglasreaktor (R1) verwendet, der einen inneren Durchmesser von 50 mm und eine Höhe von 700 mm hat. In dem Reaktor befand sich ein zentrisch angebrachter Flügelrührer mit versetzt angebrachten Flügeln auf einer Höhe von 150 mm. Der Rührer wurde über eine Stopfbuchse gegen Atmosphäre gedichtet. Die Wirbelschicht wurde extern über eine elektrische Heizung auf Reaktionstemperatur gebracht und gehalten. In den Reaktor wurde ein Strom aus Chlorethylenglykolcarbonat eingeleitet, der in einem vorgeschalteten Verdampfer (W1) nach Zugabe über eine Pumpe schonend in einem Strom aus Stickstoff erzeugt wurde. Das aus den Reaktor austretende Gas wurde durch Abkühlen kondensiert und die alle 30 Minuten aus dem Sammelbehälter (B1) entnommenen Proben wurden gaschromatographisch analysiert. Das Abgas wurde mit Hilfe eines alkalischen Wäschers (B2) HCl-frei gewaschen. Das Edukt Chlorethylenglykolcarbonat wurde mit ca. 4 g·min⁻¹ zugegeben. Zusätzlich wurde ein Inertgasstrom von 2,5 1·min⁻¹ in den Reaktor gegeben. Im Reaktor befanden sich 180 g eines mit ZnCl₂ getränkten Katalysators, hergestellt wie im oben genannten Beispiel 2 beschriebenen. Die durch Laserbeugung bestimmte mittlere Partikelgröße betrug 126 µm. Es wurde zunächst ein vollständiger Umsatz beobachtet. Die gaschromatographisch detektieren Nebenkomponenten waren im wesentlichen Essigsäure und Chloracetaldehyd, Hauptprodukt war VC. Der Umatz war mehrere Stunden stabil und sank dann langsam in Folge der Verkokung des Katalysators. Der Verlauf des Umsatzes und die Selektivitäten zu den Nebenprodukten ist in der folgenden Tabelle 1 aufgetragen.

**Tabelle 1: Verlauf von Umsatz und Nebenproduktselektivität.**

| Zeit, h | Umsatz, % | S(Essigsäure), % | S(Chloracetaldehyd), % |
|---|---|---|---|
| 0,5 | 99,9 | 1,5 | 2,7 |
| 1 | 100,0 | 0,3 | 4,6 |
| 1,5 | 100,0 | 0,2 | 4,8 |
| 2 | 99,9 | 0,2 | 6,8 |
| 2,5 | 99,9 | 0,2 | 7,0 |
| 3 | 99,9 | 0,3 | 6,4 |
| 3,5 | 99,7 | 0,3 | 6,9 |
| 4 | 99,6 | 0,2 | 7,2 |
| 4,5 | 99,4 | 0,1 | 9,3 |
| 5 | 98,9 | 0,1 | 11,8 |
| 5,5 | 98,5 | 0,1 | 12,2 |
| 6 | 98,1 | 0,1 | 12,3 |
| 6,5 | 97,3 | 0,2 | 13,0 |

Die katalytische Aktivität konnte durch Abbrennen mit Luft wieder hergestellt werden. Aufgrund der intensiven Durchmischung in der Wirbelschicht bildeten sich hierbei keine Hotspots aus, die dem Fachmann als großes Problem in der thermisch-oxidativen Regeneration von verkokten Katalysatoren bekannt sind. Die Temperatursprünge können hierbei leicht mehrer 100°C erreichen Reaktor und Katalysator schädigen. Durch die gewählte Reaktionsführung kann diese starke lokale Überhitzung vermieden werden. Bei der Regeneration mit Hilfe von Luft erwärmte sich das vorgelegte Bett nur moderat um maximal ca. 25°C.

Die durchschnittliche Ausbeute an VC bezogen auf eingesetztes CGC beträgt ca. 80% d. Th..

### Vergleichsbeispiel (Rohrreaktor, Festbett)

Der Versuchsaufbau entsprach dem in der Abb. 1 skizzierten, mit dem Unterschied, dass der Wirbelbettreaktor durch einen Rohrreaktor mit einer stationären Katalysatorschüttung ersetzt war. Das Reaktorrohr war ca. 2m lang, elektrisch thermostatisiert und hatte einen Innendurchmesser von 40mm. 2300ml = 1180g Katalysator, hergestellt wie in Beispiel 1 beschrieben, wurden eingefüllt. Die Katalysatorschüttung hatte eine Höhe von ca. 183 cm.

Entlang der Schüttungsachse waren 6 Thermoelemente angebracht, die eine Verfolgung der Innentemperatur ermöglichen.

In einem Stickstoffstrom wurde der Reaktor auf 400°C aufgewärmt und die CGC-Dosierung in den Verdampfer gestartet.

Der Katalysator wird mit 1000g CGC pro Stunde belastet, verdampft in einem Stickstoffstrom von 50-60NL / h.

**Tabelle 2: Verlauf von Umsatz und Nebenproduktselektivität.**

| Zeit, h | Umsatz, % | S(Essigsäure), % | S(Chloracetaldehyd), % |
|---|---|---|---|
| 2 | 100,0 | 0,1 | 10,3 |
| 10 | 100,0 | 0,1 | 16,0 |
| 20 | 99,9 | 0,2 | 17,8 |
| 30 | 99,8 | 0,2 | 16,8 |
| 50 | 99,2 | 0,2 | 17,3 |

Der Temperaturverlauf im Reaktorrohr und das Absinken des Umsatzes nach 50 Stunden zeigte, dass die Katalysatorschüttung regeneriert werden musste.

Zu diesem Zeitpunkt war VC mit einer durchschnittlichen Ausbeute von 69% d. Th. erhalten worden.

Zur Regenerierung wurde die Eduktdosierung gestoppt und ein Gasgemisch aus 30NL Luft und 60NL Stickstoff pro Stunde über den Kontakt geleitet. Nach 3 Stunden Abbrennen wurde die Luftmenge auf 60NL erhöht.

Nach 50 Minuten stieg die Katalysatortemperatur am vorletzten Thermofühler plötzlich von 400 auf 480°C mit steigender Tendenz an, worauf die Luftzufuhr kurz unterbrochen, und nachdem sich die Katalysatortemperatur normalisiert hatte, die Luftmenge wieder auf 30NL/h eingestellt wurde.

Nach weiteren 13h Abbrennen wurde die Luftmenge wieder auf 60NL/h erhöht, worauf nach kurzer Zeit im vorderen Teil der Katalysatorschüttung, an der 3. Messstelle ein 511°C heißer HOT SPOT angezeigt wurde.

Die Luft zufuhr wurde wieder kurz unterbrochen und dann 4h bei 30 und 9 h 40 NL Luft weiter abgebrannt. Plötzlich bildete sich an der 2. Messstelle eine HOT SPOT, der durch Abstellen der Luft an einem weiteren Anwachsen der Temperatur gehindert wurde.

Nach weiteren 9h bei 30 NL/h wurde in der Annahme der überwiegende Teil der Ablagerungen sei nun abgebrannt auf 40 NL/Luft gestellt und der zusätzliche Stickstoffstrom abgestellt.

Nach 2,5h Abbrennen im reinen Luftstrom bildete sich plötzlich an der 1. Temperaturmessstelle ein HOT SPOT mit 711 °C aus, der durch sofortiges Umstellen auf reinen Stickstoff abgefangen wurde.

Der Katalysator wurde noch weitere 3 Stunden unter intensiver Beobachtung mit 200 NL Luft abgebrannt. Dann wurde wieder, wie oben beschrieben, CGC in einem heißen Stickstoffsrom auf den Kontakt gefahren.

Die gesamte Regenerationsdauer betrug 45 Stunden

Der Katalysator zeigte wieder die alte Aktivität, in den nächsten Läufen wurde schon nach 24 h abgebrannt, damit sich nicht so viel kohlenstoffreiche Ablagerungen auf dem Katalysator abscheiden.

Der Stickstoffstrom zum Regenerieren wurde auf das bis zu 10 fache erhöht.

Das Abbrennverhalten wurde durch diese Maßnahmen deutlich stabiler, ein Scale Up in den technischen Maßstab erscheint jedoch als riskant.

Der Katalysator wurde so 15 Zyklen mit ca. 332 h Produktionszeit und 181 h Regenerationszeit betrieben.

Die durchschnittlichen Ausbeute an VC über den gesamten Prozess war stabil ca. 69 % d. Th..

## Patentansprüche

1. Ein Verfahren zur Herstellung von Vinylencarbonat durch Abspaltung von Chlorwasserstoff aus Chlorethylenglykolcarbonat (CGC) an festen Kontakten in der Gasphase, **dadurch gekennzeichnet, dass** die Umsetzung zwischen 300 und 600°C an fluidisierten Kontaktkatalysatoren in einem Wirbelbett gegebenenfalls unter Inertgas, durchgeführt wird wobei die Katalysatorschüttungen aus einem inerten Trägermaterial mit aufgebrachten Metallen der I. oder II. Nebengruppe oder der VIII. Nebengruppe des Periodensystems der Elemente oder die Katalysatorschüttungen ganz aus den Salzen oder Oxyden der Metalle der I., II. oder VIII. Nebengruppe des Periodensystems der Elemente bestehen.

2. Verfahren nach Anspruch 1, **dadurch gekennzeichnet, dass** die Umsetzung zwischen 350 und 500°C durchgeführt wird.

3. Verfahren nach Anspruch 1, **dadurch gekennzeichnet, dass** das Inertgas ein Gas aus der Gruppe Helium, Argon, Neon, Stickstoff, Kohlenmonoxid oder Kohlendioxid ist.

4. Verfahren nach Anspruch 1, **dadurch gekennzeichnet, dass** die Katalysatorschüttungen aus Chloriden der Metalle der I. und II. Nebengruppe oder der VIII. Nebengruppe des Periodensystems der Elemente bestehen.

5. Verfahren nach Anspruch 1, **dadurch gekennzeichnet, dass** die Katalysatorschüttungen aus Zinkoxid und/oder Zinkchlorid oder aus einem inerten Trägermaterial mit aufgebrachten Zinkoxid und/oder Zinkchlorid bestehen.

## Claims

1. Process for the production of vinylene carbonate by eliminating hydrogen chloride from chloroethylene glycol carbonate (CGC) over solid catalysts in the gas phase, **characterized in that** the reaction is optionally carried out under inert gas at between 300 and 600°C over fluidized contact catalysts in a fluidized bed, the catalyst beds consisting of an inert support material with applied metals of subgroup I, II or VIII of the Periodic Table of the Elements or the catalyst beds consisting completely of the salts or oxides of metals of subgroup I, II or VIII of the Periodic Table of the Elements.

2. Process according to Claim 1, **characterized in that** the reaction is carried out at between 350 and 500°C.

3. Process according to Claim 1, **characterized in that** the inert gas is a gas from the group consisting of helium, argon, neon, nitrogen, carbon monoxide and carbon dioxide.

4. Process according to Claim 1, **characterized in that** the catalyst beds consist of chlorides of metals of subgroup I, II or VIII of the Periodic Table of the Elements.

5. Process according to Claim 1, **characterized in that** the catalyst mixtures consist of zinc oxide and/or zinc chloride or of an inert support material with applied zinc oxide and/or zinc chloride.

## Revendications

1. Procédé de fabrication de carbonate de vinylène par clivage de chlorure d'hydrogène à partir de carbonate de chloroéthylène-glycol (CGC) sur des contacts solides en phase gazeuse, **caractérisé en ce que** la réaction est réalisée entre 300 et 600 °C sur des catalyseurs de contact fluidisés dans un lit fluidisé, éventuellement sous un gaz inerte, les garnissages catalytiques étant constitués d'un matériau support inerte avec des métaux appliqués du groupe de transition I ou II ou du groupe de transition VIII du tableau périodique des éléments ou l'ensemble des garnissages catalytiques étant constitué des sels ou oxydes des métaux du groupe de transition I, II ou VIII du tableau périodique des éléments.

2. Procédé selon la revendication 1, **caractérisé en ce que** la réaction est réalisée entre 350 et 500°C.

3. Procédé selon la revendication 1, **caractérisé en ce que** le gaz inerte est un gaz du groupe hélium, argon, néon, azote, monoxyde de carbone ou dioxyde de carbone.

4. Procédé selon la revendication 1, **caractérisé en ce que** les garnissages catalytiques sont constitués de chlorures des métaux du groupe de transition I et II ou du groupe de transition VIII du tableau périodique des éléments.

5. Procédé selon la revendication 1, **caractérisé en ce que** les garnissages catalytiques sont constitués d'oxyde de zinc et/ou de chlorure de zinc ou d'un matériau support inerte avec de l'oxyde de zinc et/ou du chlorure de zinc appliqués.
